# EUROPÄISCHE PATENTANMELDUNG

(11) **EP 2 517 571 A2**
(43) Veröffentlichungstag der Anmeldung: **31.10.2012**
(21) Anmeldenummer: 12158896.6
(22) Anmeldetag: 09.03.2012
(51) Int. Cl.: A23L 1/00, A23L 1/39

(54) **Würzschaum sowie Verfahren und Zusammensetzung zu dessen Herstellung**

(30) Priorität: 07.07.2011 DE 102011106881; 09.03.2011 DE 102011013447
(71) Anmelder: Fraunhofer-Gesellschaft zur Förderung der angewandten Forschung e.V., 80686 München (DE)
(72) Erfinder: Bruzzano, Stefano, 47051 Duisburg (DE); Pfeifer, Sebastian, 47055 Duisburg (DE); Somborn-Schulz, Annette, 45661 Recklinghausen (DE); Bretz, Karlheinz, 46049 Oberhausen (DE); Rettweiler, Manuela, 46047 Oberhausen (DE)

(57) **Zusammenfassung**

Es wird ein Verfahren zur Herstellung eines Würzschaums beschrieben, wobei der Würzschaum ein Protein sowie ein Fett und/oder ein Wachs umfasst. Das Verfahren umfasst folgende Schritte:
A) Bereitstellung der Fettsäureesterkomponente und der Proteinkomponente, wobei
- die Fettsäureesterkomponente und/oder die Proteinkomponente einen schaumstabilisierenden Bestandteil enthalten
- zumindest die Fettsäureesterkomponente im Wesentlichen biogenen Ursprungs ist,
- die Proteinkomponente und die Fettsäureesterkomponente in einem Gewichtsverhältnis von 1:10 bis 9:1 vorliegen,
- in die Fettsäureesterkomponente mindestens ein Würzstoff eingebracht ist oder eingebracht wird,

B) Mischen der Proteinkomponente und der Fettsäureesterkomponente gegebenenfalls, so dass enthaltenes Wasser und die summierten Anteile von Proteinkomponente und Fettsäureesterkomponente in einem Gewichtsverhältnis von 1:1 bis 20:1 vorliegen und wobei der Anteil der Fettsäureesterkomponente im entstandenen Gemisch größer 10 Gew.-% ist,
C) Einbringen eines Gases in das nach Schritt B) erhaltene Gemisch, so dass ein Schaum entsteht.

## Beschreibung

Die Erfindung betrifft ein Verfahren zur Herstellung eines Würzschaums, der zum einen eine Fettsäureesterkomponente, also ein Fett und/oder ein Wachs, und zum anderen ein Protein umfasst. Die Ausgangsmaterialien sind dabei im Wesentlichen biogenen Ursprungs und stellen in Form eines wässrigen Gemischs eine aufschäumbare Zusammensetzung dar. Zur Herstellung eines Schaums wird in diese Zusammensetzung ein Gas eingebracht.

Nach dem Stand der Technik sind eine Reihe von Lebensmittelschäumen bekannt, bei denen eine biogene Substanz, die sowohl eine Fettsäureesterkomponente als auch ein Protein umfasst, als solche aufgeschäumt wird (z.B. Sahne zu Schlagsahne). Im Rahmen der vorliegenden Anmeldung werden allerdings keine Würzschäume beschrieben, bei denen die Fettsäureesterkomponente und das Protein bereits in derselben biogenen Substanz vorliegen.

Bei der Herstellung von Lebensmittelschäumen werden nach dem Stand der Technik unter Umständen synthetische Tenside auf Grund ihrer schaumbildenden bzw. emulgierenden Eigenschaften zugesetzt. Diese haben aber den Nachteil, dass sie zu Unverträglichkeiten führen können.

Eine Aufgabe der Erfindung ist es, einen verträglichen Würzschaum und ein Verfahren zu dessen Herstellung anzugeben.

Diese Aufgabe wird durch das Verfahren zur Herstellung eines Würzschaums gemäß dem Hauptanspruch und den Würzschaum bzw. die Zusammensetzung zu dessen Herstellung gemäß den unabhängigen Ansprüchen gelöst. Weitere Ausgestaltungen und Weiterbildungen sind Gegenstand von Unteransprüchen und gehen weiterhin aus der nachfolgenden Beschreibung hervor.

Ein Verfahren zur Herstellung eines Würzschaums, der zum einen ein Protein und zum anderen eine Fettsäureesterkomponente (also ein Fett und/oder ein Wachs) umfasst, weist die folgenden Schritte auf:
A) Zunächst wird die Fettsäureester und die Proteinkomponente bereit gestellt; die Fettsäureesterkomponente und/oder die Proteinkomponente werden dabei so ausgewählt, dass sie einen schaumstabilisierenden Bestandteil enthalten, wobei entweder die Proteinkomponente ein schaumstabilisierendes Protein enthält oder die Fettsäureesterkomponente einen schaumstabilisierenden Bestandteil enthält oder sowohl die Proteinkomponente als auch die Fettsäureesterkomponente einen schaumstabilisierenden Bestandteil enthält, nämlich ein schaumstabilisierendes Protein bzw. einen schaumstabilisierenden Fettsäureester-Bestandteil. Alternativ kann die Proteinkomponente aus dem schaumstabilisierenden Protein und/oder die Fettsäureesterkomponente aus dem schaumstabilisierenden Fettsäureester-Bestandteil bestehen. In die Fettsäureesterkomponente wird ferner die Würzstoffkomponente eingebracht oder ist bereits eingebracht. Ferner werden die Fettsäureesterkomponente und die Proteinkomponente so ausgewählt, dass das Gewichtsverhältnis Fettsäureesterkomponente : Proteinkomponente 1 : 9 bis 10 : 1 betragen kann (das Gewichtsverhältnis bezieht sich hierbei auf die Fettsäureesterkomponente ohne darin eingebrachten Würzstoff); bei der Proteinkomponente ist ferner zu beachten, dass wie generell im Rahmen dieser Anmeldung gilt, für die Bestimmungen des Anteils in Gewichtsprozent die wässrigen Bestandteile, die ggf. in einer Proteinkomponente enthalten sein können, nicht in den Anteil der Proteinkomponente mit eingerechnet sind. Wesentlich bei der Auswahl der Fettsäureesterkomponente und der Proteinkomponente ist, dass zumindest die Fettsäureesterkomponente, und gegebenenfalls auch die Proteinkomponente und der schaumstabilisierende Bestandteil im Wesentlichen biogenen Ursprungs sind.
B) In einem nachfolgenden Schritt werden die Proteinkomponente und die Fettsäureesterkomponente gemischt, wobei gegebenenfalls Wasser zugesetzt wird (insbesondere, wenn die Proteinkomponente nicht als wässriges Gemisch eingesetzt wird oder zu wenig Wasser bezüglich der Wassermenge, die für die herzustellende Zusammensetzung erforderlich ist, vorhanden ist). Das Wasser einerseits und die aufsummierten Anteile von Proteinkomponente und Fettsäureesterkomponente andererseits liegen in einem Gewichtsverhältnis von 1 : 1 bis 20 : 1 vor (das Wasser liegt also im Regelfall im Überschuss vor). Das dabei erhaltene Gemisch weist einen Anteil der Fettsäureesterkomponente auf, der größer als 10 Gew.-% ist (bezogen auf die summierten Anteile von Wasser, Fettsäureesterkomponente, Proteinkomponente und Würzstoff).
C) In das in Schritt B) erhaltene Gemisch wird schließlich ein Gas eingebracht, so dass ein Schaum entsteht.

Mit dem erfindungsgemäßen Verfahren kann ein Würzschaum hergestellt werden, der gut dosierbar ist, was insbesondere aus dem hohen Gasvolumen im Verhältnis zum nicht gasförmigen Volumen (nachfolgend auch "Flüssigkeitsvolumen" bezeichnet) resultiert. Die Würzstoffe lassen sich also gezielt niedrig dosiert und homogen verteilen. Erfindungsgemäß dienen die Fettsäureesterkomponente und/oder die Proteinkomponente zur Schaumstabilisierung, so dass auf synthetische Tenside gänzlich verzichtet werden kann.

Auf Grund der Verwendung der Fettsäureesterkomponente bzw. der Proteinkomponente treten gänzlich neue Eigenschaften auf. Die erfindungsgemäßen Schäume fühlen sich feucht an und glänzen.

Ferner ist es auf Grund des relativ großen Ölanteils im Würzschaum sehr leicht möglich, die Würzstoffe in die zu würzende Speise einzubringen. Die Fettsäureesterkomponente dient als Träger- und/oder Transportsystem für die Würzstoffe, insbesondere für lipophile Würzstoffe.

Im Vergleich zu reinen Ölen weisen die erfindungsgemäßen Schäume den Vorteil auf, dass sie wesentlich besser handhabbar bei der Applikation sind, was nicht nur auf der besseren Dosierbarkeit beruht, sondern auch darauf, dass kein oder nur ein geringes Tropfen der aufgebrachten Masse erfolgt.

Gemäß einer Ausführungsform bildet das in Schritt B) erhaltene Gemisch eine Emulsion aus. Insbesondere liegt dabei die zumindest die Fettsäureesterkomponente im Wasser als disperse Phase vor, insbesondere in Form von Tröpfchen. Prinzipiell ist aber auch denkbar, dass Wasser die disperse Phase ist. Der mittlere Teilchendurchmesser der dispersen Phase beträgt im Regelfall 20 nm bis 50 µm (gemessen mittels dynamischer Lichtstreuung), insbesondere 100 nm bis 10 µm. Es kann sich dabei beispielsweise um eine Emulsion mit einem mittleren Teilchendurchmesser größer 200nm (vergleichbar mit einer Makroemulsion), eine Emulsion mit einem mittleren Teilchendurchmesser von 100 nm bis 200nm (vergleichbar mit einer Miniemulsion) oder um eine transparente Emulsion, insbesondere mit einem mittleren Teilchendurchmesser von kleiner 100 nm (vergleichbar mit einer Mikroemulsion) handeln. Wie bereits vorstehend ausgeführt wurde, kann bei der nach Schritt B) erhaltenen Zusammensetzung auf synthetische Tenside verzichtet werden. Liegt diese nun als Emulsion vor, so bedeutet dies auch, dass bei dieser Emulsion auf synthetische Emulgatoren verzichtet werden kann.

Zumindest die erfindungsgemäß bereitgestellte Fettsäureesterkomponente ist im Wesentlichen biogenen Ursprungs. Gemäß einer Ausführungsform sind auch die Proteinkomponente und der schaumstabilisierende Bestandteil im Wesentlichen biogenen Ursprungs.

Unter "biogenem Ursprung" ist "biologischen oder organischen Ursprungs" zu verstehen, der Begriff umfasst also nicht Materialien chemisch-synthetischer Herkunft. Der Begriff "Fettsäureesterkomponente, Proteinkomponente und schaumstabilisierender Bestandteil biogenen Ursprungs" umfasst somit alle die Fettsäureesterkomponenten, Proteinkomponenten und schaumstabilisierende Bestandteile, die von Pflanzen, Tieren oder Mikroorganismen (insbesondere Pilzen, Algen und Bakterien) gebildet werden.

"Im Wesentlichen" biogenen Ursprungs bedeutet dabei, dass zumindest 90 Gew.-% der Fettsäureesterkomponente, meist mehr als 95 Gew.-% und häufig mehr als 99 Gew.-% biogenen Ursprungs sind. Idealerweise sind 100 Gew.-% der Fettsäureesterkomponente 100 % biogenen Ursprungs.

Ist auch die Proteinkomponente biogenen Ursprungs, so gilt hierbei im Regelfall, dass sowohl 90 Gew.-% der Fettsäureesterkomponente als auch 90 Gew.-% der Proteinkomponente biogenen Ursprungs sind. Üblicherweise ist der Anteil beider Komponenten aber zu mehr als 95 % biogenen Ursprungs, häufig zu mehr als 99 Gew.-% biogenen Ursprungs. Idealerweise sind sowohl die Fettsäureesterkomponente als auch die Proteinkomponente zu 100 % biogenen Ursprungs. Entsprechend gilt für den schaumstabilisierenden Bestandteil, der zumindest teilweise in der Fettsäureesterkomponente und/oder der Proteinkomponente enthalten ist, dass dieser - unabhängig von den vorstehend angegebenen Zahlen für Fettsäureesterkomponente und Proteinkomponente - zumindest zu 90 % biogenen Ursprungs ist, im Regelfall zumindest zu 99 % biogenen Ursprungs ist und häufig zu 100 % biogenen Ursprungs ist.

Unter biogenem Ursprung kann darüber hinaus gemäß einer Ausführungsform auch verstanden werden, dass synthetisch hergestellte Materialien wie beispielsweise synthetisch hergestellte Triglyceride aus den natürlich vorkommenden Ölen enthaltenen Fettsäuren und unverseifbare Bestandteile von natürlichen Ölen (beispielsweise Squalen), die synthetisch hergestellt sind, so miteinander kombiniert werden, dass eine Zusammensetzung entsteht, die den erfindungsgemäß beschriebenen Materialien, die im Wesentlichen biogenen Ursprungs sind, entsprechen.

Gemäß einer Ausführungsform werden als im Wesentlichen biogene Materialien Materialien zugesetzt, die für die Lebensmittelindustrie zugelassen sind. Dies gilt unabhängig voneinander für die Fettsäureesterkomponente und die Proteinkomponente, bevorzugt gilt es für beide Komponenten. Beide Komponenten können unabhängig voneinander pflanzlichen und/oder tierischen Ursprungs sein.

Die erfindungsgemäß eingesetzte Fettsäureesterkomponente kann ein Fett sein, also ein Material, das im Wesentlichen aus Mono-, Di- und Triglyceriden besteht, wobei der Anteil von Mono- und Diglyceriden gegenüber den von Triglyceriden meist sehr gering ist (also meist < 5 Gew.-% der Gesamtglyceridmenge). Neben den Glyceriden sind im Regelfall noch unverseifbare Bestandteile in den Fetten enthalten, deren Anteil meist < 5 Gew.-% in der Fettsäureesterkomponente beträgt, aber im Einzelfall auch deutlich darüber liegen kann.

Gemäß einer Ausführungsform kann die Fettsäureesterkomponente aus Pflanzenölen bestehen oder diese umfassen. Das Pflanzenöl kann dabei ausgewählt sein aus einem oder mehreren der nachfolgend beispielhaft genannten Öle: Amaranthöl, Aprikosenkernöl, Arganöl, Avellanaöl, Avocadoöl, Babassuöl, Baobaböl, Borretschsamenöl, Broccolisamenöl, Cupuaçuöl, Distelöl, Granatapfelsamenöl, Hanföl, Erdnussöl, Haselnussöl, Holundersamenöl, Jatrophaöl, Johannisbeersamenöl, Kakaobutter, Kameliensamenöl, Kokosöl, Kürbiskernöl, Macadamianussöl, Mandelöl, Maiskeimöl, Marulaöl, Nachtkerzenöl, Olivenöl, Preiselbeersamenöl, Reiskeimöl, Rapsöl, Reisöl, Sanddornöl, Schwarzkümmelöl, Sesamöl, Sheabutter, Sheanussöl, Sojaöl, Sonnenblumenöl, Traubenkernöl, Palmöl, Palmkernöl, Walnusskernöl, Weizenkeimöl, Wiesenschaumkrautöl und Wildrosenöl. Generell kann also ein beliebiges aus Pflanzen herstellbares Öl ausgewählt werden, wobei dann häufig als Bestandteile der Triglyceride Ölsäure und Palmitinsäure enthalten sind und häufig auch noch zusätzlich Linolsäure und/oder Stearinsäure.

Als geeignet haben sich insbesondere Öle erwiesen, die Lecithin enthalten, insbesondere in einem Anteil von zumindest 0,25 Gew.-%, insbesondere von 0,5 Gew.-%, zu nennen sind beispielsweise Sonnenblumenöl, Sojaöl, Weizenkeimöl oder Sesamöl. Ferner können als Öle auch solche Öle eingesetzt werden, die zumindest einen der folgenden Bestandteile enthalten: Vitamine, Mineralstoffe, Karotinoide, Phytosterole und Flavonoide. Die zuletzt genannten Bestandteile sind dann anmeldungsgemäß nicht als Würzstoffe anzusehen sondern ausschließlich als Bestandteil der Fettsäureesterkomponente.

Bei der Auswahl der Öle ist gegebenenfalls auf die Stabilität, insbesondere Oxidationsstabilität zu achten. Bestimmte Öle (z.B. Leinöl) werden bei Luftkontakt relativ schnell ranzig, so dass bei Verwendung derartiger Öle die aufschäumbare Zusammensetzung unter Luftausschluss und möglichst auch unter Lichtausschluss z.B. in einer Druckgasdose gelagert werden sollte und erst direkt vor der Applikation aufgeschäumt werden sollte.

Neben Fetten bzw. Ölen kommen als Fettsäureesterkomponente insbesondere auch Wachse in Betracht. Hierbei handelt es sich um Materialien, die auf Estern von Fettsäuren basieren, wobei insbesondere einwertige Alkohole und nicht Glycerine als Alkoholkomponente fungieren. Der Anteil der Fettsäureesterkomponente beträgt üblicherweise zumindest 70 %; daneben können beispielsweise Paraffinkohlenwasserstoffe und andere unverseifbare Komponenten vorliegen. Als Wachse können beispielsweise Bienenwachs, Beerenwachs, Japanwachs, Jojobaöl, Wollwachs, Carnaubawachs und Zuckerrohrwachs genannt werden. Es können selbstverständlich auch Gemische dieser Wachse verwendet werden. Generell können also Wachse pflanzlicher und/oder tierischer Herkunft eingesetzt werden.

Selbstverständlich können auch Gemische einer oder mehrerer Fette und einer oder mehrerer Wachse bereitgestellt werden.

Gemäß meiner Ausführungsform der Erfindung wird als Fettsäureesterkomponente ein Öl oder ein Wachs bereitgestellt, dass bei 37° Celsius flüssig ist. Dies hat den Vorteil, dass die Fettsäureesterkomponente beim Auftragen auf die zu würzende Speise in flüssigem Zustand vorliegen kann ohne, dass ein zu starkes Erwärmen der Speise erforderlich ist und dementsprechend ein gutes Einwirken der Fettsäureesterkomponente und der darin enthaltenen Würzstoffe möglich ist. Der Würzschaum kann aber auch eine Fettsäureesterkomponente enthalten, die schon bei Raumtemperatur (21°C) oder gegebenenfalls sogar bei der in Kühlräumen vorherrschenden Temperatur flüssig ist.

Gemäß einer weiteren Ausführungsform der Erfindung beträgt der Gewichtsanteil der Fettsäureesterkomponente des in Schritt B) erhaltenen Gemischs 10 bis 50 Gew.-%, insbesondere 10 bis 35 Gew.-%. Beispielsweise kann der Gewichtsanteil der Fettsäureesterkomponente 15 bis 20 Gew.-% betragen. Der Gewichtsanteil der Fettsäureesterkomponente ist also relativ hoch, was erfindungsgemäß erwünscht ist, um eine Rückfettung zu bewirken. Ferner kann bei einem hohen Fettsäureesteranteil auch eine große Menge lipophiler Würzstoffe eingebracht werden.

Die erfindungsgemäße Proteinkomponente kann insbesondere ein Protein und/oder ein Proteinextrakt umfassen. Im Einzelfall kann als Proteinkomponente auch ein reines Protein bzw. ein Gemisch von mehreren der genannten Materialien eingesetzt werden; die Proteinkomponente besteht dann aus einem Protein und/oder einem Proteinextrakt. Da allerdings vorzugsweise biogene Materialien eingesetzt werden, enthält die Proteinkomponente neben dem Protein bzw. dem Proteinextrakt in den allermeisten Fällen auch noch weitere Stoffe. Der Proteingehalt in der Proteinkomponente beträgt üblicherweise mehr als 30 Gew.-% und in der Regel auch mehr als 50 Gew.-%. Häufig ist der Proteingehalt allerdings noch höher, insbesondere > 70 Gew.-%. Beispielsweise können die Proteingehalte auch durch Entölen und ähnliches stärker erhöht werden; insbesondere in diesen Fällen können auch Proteingehalte > 80 Gew.-% und sogar > 90 Gew.-% erreicht werden. Der Proteingehalt ist dabei definiert als der Gehalt, der sich aus der Bestimmung des Stickstoffs und dessen Multiplikation mit dem Faktor 6,25 errechnet. Der Stickstoffgehalt wird dabei mittels herkömmlicher Elementaranalyse ermittelt. Unter Protein wird erfindungsgemäß jegliches Polypeptid mit zumindest zwei Aminosäureeinheiten verstanden.

Der Anteil der Proteinkomponente in dem Gemisch, das nach Schritt B) erhalten wird beträgt üblicherweise zwischen 5 und 25 Gew.-%, insbesondere zwischen 10 und 20 Gew.-%.

Die Proteine bzw. Proteinextrakte können sowohl pflanzlichen als auch tierischen Ursprungs sein. Als tierische Proteine sind insbesondere Ei, beispielsweise Hühnerei-Proteine, Milch-Proteine und Gelatine-Proteine (z. B. aus Schlachtabfällen) und deren Extrakte, die beispielsweise in Form von Hydrolysaten vorliegen können, zu nennen. Als pflanzliche Proteine sind insbesondere Getreide-Proteine (z. B. Weizenkleber-Präparate), Hülsenfrucht-Proteine (z. B. Soja-Proteine, Erbsen-Proteine oder Lupinen-Proteine) oder auch Proteine aus Samen (z. B. Rapssamen oder Sonnenblumenkerne) sowie Proteine aus Knollenpflanzen (z. B. Kartoffel-Proteine) zu nennen.

Zur Proteinextraktion können beispielsweise die pflanzlichen oder tierischen Ausgangsstoffe zunächst entölt werden (etwa mit lipophilen Lösungsmitteln). Auch eine alternative oder zusätzliche Flockierung oder ein Pressen der pflanzlichen und tierischen Ausgangsmaterialien ist denkbar. Durch die Extraktion kann beispielsweise der Restölgehalt in der Proteinkomponente auf ≤ 5 Gew.-% reduziert werden. Ferner kann eine Proteinextraktion mittels Walzentrocknung erfolgen.

Als Proteinkomponente können aber auch bereits aus den Naturstoffen isolierte Proteine eingesetzt werden. Hier sind beispielsweise zu nennen Albumine (tierisch), Globulin (tierisch), Glutelin (Getreide), Histone, Protamine (tierisch), konjugierte Proteine wie Phosphoproteine, Chromoproteine, Lecithoproteine, Peptone. Erfindungsgemäß können aber auch Proteinkomponenten eingesetzt werden, die diese Proteine neben anderen Stoffen enthalten.

Wie ausgeführt, kann die Proteinkomponente einen Restfettgehalt aufweisen. Im Regelfall liegt dieser Restfettgehalt allerdings unter 10 Gew.-%, so dass beim Fettgehalt des gesamten Schaums der Fettgehalt des Proteins gegenüber dem Gehalte der Fettsäureester-Komponente eine eher untergeordnete Rolle spielt. Häufig ist der Restfettgehalt der Proteinkomponente sogar geringer als 5 Gew.-%. Am fertigen Würzschaum kann dies auch daran erkannt werden, dass die im Gesamtgemisch enthaltenen Fettsäuren im Wesentlichen denen der Fettsäureesterkomponente entsprechen. Zudem wird häufig die Fettsäureesterkomponente und die Proteinkomponente nicht denselben pflanzlichen bzw. tierischen Ursprung haben, sondern aus unterschiedlichen Pflanzenarten bzw. Tieren gewonnen sein. Insbesondere sind die in Schritt A) bereitsgestellte Fettsäureesterkomponente und Proteinkomponente nicht zueinander identisch und ergeben auch nach Schritt B) keine Zusammensetzung, die als solche nicht unter dem Begriff eines "biogenen" Ausgangsmaterials gemäß der vorliegenden Anmeldung, wie es in Schritt A) für die Proteinkomponente oder die Fettsäureesterkomponente verwendet werden kann, subsummiert werden kann. Wird beispielsweise ein pflanzliches Öl als Fettsäureesterkomponente verwendet und ein Milchprotein oder ein Trockenmilchpulver als Proteinkomponente, so ist ein Fettgehalt des Würzschaums der Anteil, der auf die Milch-Komponente zurückgeht im Regelfall schon daran festzumachen, dass Milch einen sehr viel höheren Anteil an kurzkettigen "Fettsäuren" mit vier bis fünfzehn Kohlenstoffatomen enthält, der bis zu 35 Gew.-% im Milchfett betragen kann.

Als Proteinkomponente kann schließlich auch neben den beispielsweise als Hydrolysat vorliegenden Proteinextrakten der Einsatz modifizierter Proteine erfolgen. Die in der Proteinkomponente enthaltenen Proteine können also auch - müssen aber nicht - chemisch modifiziert sein, insbesondere acetyliert, verestert, desaminiert und/oder amidiert. Die chemische Modifikation kann beispielsweise biochemisch, insbesondere enzymatisch, aber grundsätzlich auch mittels organisch/anorganisch-chemischer Reagenzien erfolgen. Diese modifizierten Proteine sind gemäß einer Ausführungsform dieser Anmeldung, zumindest, wenn es sich um biochemische Modifikationen eines Proteins handelt, ebenfalls als biogenen Ursprungs anzusehen, wenn das Proteingrundgerüst nicht synthetisch aufgebaut wurde sondern aus einem biogenen Material erhalten wurde, da lediglich im geringen Ausmaß eine chemische Modifikation der vorhandenen funktionellen Gruppen des Proteins erfolgt.

Gemäß einer Ausführungsform weisen zumindest 75 Gew.-% der in der Proteinkomponente enthaltenen Proteine ein Molekulargewicht ≤ 100 kDa, insbesondere ≤ 10 kDa, auf, im Regelfall werden sogar mehr als 90 Gew.-% der in der Proteinkomponente enthaltenen Proteine unter einer oder beiden der genannten Schranken liegen und häufig auch mehr als 95 Gew.-% unter einer oder beiden der genannten Schranken. Eine derartige Reduzierung auf eher kürzerkettige Proteine hat den Vorteil, dass bei Anwendungen, die die Applikation des Würzschaums auf eine zu würzende Speise, kein Abrieb entstehen sollte, der sich bei größeren Molekülmassen bilden würde und optisch ein unerwünschtes Erscheinungsbild des applizierten Schaums hinterlässt.

Der wesentlichste Bestandteil der erfindungsgemäßen Würzschäume ist der schaumstabilisierende Bestandteil. Unter einem schaumstabilisierenden Bestandteil wird dabei verstanden, dass in der Schaumzusammensetzung Moleküle oder Molekülteile enthalten sind, die die Bildung von Gasblasen stabilisieren. Der schaumstabilisierende Bestandteil ist erfindungsgemäß insbesondere in der Fettsäureesterkomponente und/oder der Proteinkomponente enthalten; andere Bestandteile des Würzschaums (abgesehen von Wasser) spielen im Regelfall nur eine untergeordnete Rolle für die Stabilisierung des Schaums, so dass häufig der schaumstabilisierende Bestandteil ausschließlich in der Fettsäureesterkomponente und/oder der Proteinkomponente enthalten ist (wenn man von eventuell durch den Würzstoff auftretenden Effekten absieht - wobei die primäre Aufgabe des Würzstoffes aber nicht die Schaumstabilisierung sondern die spezifische Verwendung des Schaums ist).

In den meisten Fällen wird der schaumstabilisierende Bestandteil entweder nur durch die Proteinkomponente (bzw. eines Teils desselben) oder durch Fettsäureesterkomponente und Proteinkomponente zusammen zur Verfügung gestellt. Allerdings sind auch fettstabilisierte Schäume prinzipiell denkbar, insbesondere wenn ein Teil des Fettes in kristalliner Form vorliegt. Im Regelfall sind die erfindungsgemäßen Schäume aber stark proteinstabilisiert, wobei der schaumstabilisierende Proteinbestandteil bzw. das schaumstabilisierende Protein dann Aminosäuren bzw. Aminosäuresequenzen aufweist, durch die insbesondere bei der Bildung des Schaums die Grenzfläche zur Luft bzw. dem Gas stabilisiert wird. Die Proteinstruktur des schaumstabilisierenden Proteins weist daher eine entsprechend ausgewogene Anzahl und Anordnung von hydrophoben und hydrophilen Aminosäuren auf, wie sie beispielsweise in Peptiden wie β-Lactoglobulin, α-Lactalbumin und β-Casein vorhanden ist. Als schaumstabilisierende Proteine sind ferner zu nennen: Ei-Albumin, Casein-Hydrolysate, Keratin-Hydrolysate, Soja-Peptones und Soja-Albumin, Molke und Milchserumproteine, Weizenalbumin, Weizenglobulin, Conalbumin und Avidin.

Als schaumstabilisierender Bestandteil der Fettsäureesterkomponente ist beispielsweise Lecithin zu nennen.

Zur Verbesserung der schaumstabilisierenden Eigenschaften der Proteinkomponente kann das proteinhaltige Ausgangsmaterial auch so behandelt werden, dass die schaumstabilisierenden Segmente oder Proteine gezielt angereichert werden. Beispielsweise kann eine enzymatische Hydrolyse des proteinhaltigen Ausgangsmaterials erfolgen, wobei bei niedrigen Hydrolysegraden häufig die Grenzflächeneigenschaften des Proteins ein Maximum annehmen. Ferner kann eine gezielte Fraktionierung des Rohstoffs durchgeführt werden, beispielsweise mittels Filtrationsverfahren oder spezielle Fällungsstrategien. Verbesserte Grenzflächeneigenschaften werden dabei insbesondere in den niedermolekularen Fraktionen erhalten. Beispielsweise können mittels Ultrafiltration Proteine mit einem Molekulargewicht von 1 bis 200 kDa erhalten werden.

Durch die genannten Verfahren ist es somit auch möglich, bei einem gegebenen Proteinausgangsmaterial nicht nur die Schaumstabilisierung zu erhöhen, sondern auch die Molekülgröße so zu erniedrigen, dass beim Auftragen des proteinhaltigen Schaums auf der Haut ein möglichst geringer Abrieb entsteht.

Gemäß einer Ausführungsform ist der Würzstoff derart in die Fettsäureester eingebracht, dass er in gelöster Form vorliegt, insbesondere vollständig gelöst, oder in verkapselter Form vorliegt. Dies hat den Vorteil, dass eine besonders homogene Verteilung des Würzstoffs in der Fettsäureesterkomponente und damit auch im Würzschaum gewährleistet werden kann. Eine verkapselte Form des Würzstoffs bietet sich insbesondere dann an, wenn der Würzstoff an sich nicht besonders lipophil ist und mit einer Hülle die Lipophilie erhöht werden kann. Ferner ist eine Verkapselung z. B. dann sinnvoll, wenn der Würzstoff durch die Verkapselung eine verbesserte Stabilität besitzt, etwa weil er flüchtig ist oder sich in unverkapselter Form in unerwünschter Weise verändert (etwa durch Reaktion mit Luftsauerstoff).

Der Würzstoff kann bereits in der biogenen Fettsäureesterkomponente und/oder der biogenen Proteinkomponente enthalten sein; im Regelfall wird der Würzstoff aber erst während des erfindungsgemäßen Verfahrens zugesetzt. Ferner kann der Würzstoff flüssig sein, im Regelfall handelt es sich aber um einen Feststoff.

Gemäß einer Ausführungsform ist der Würzstoff ausgewählt aus Raucharomen, Pflanzen oder Pflanzenteilen, tierischen Substanzen sowie Gemischen der vorgenannten Würzstoffklassen. Im Einzelfall können alternativ oder zusätzlich auch synthetische Aromen (naturidentische oder synthetische Aromen) als Würzstoff vorliegen. Als Pflanzen bzw. Pflanzenteile werden hierbei insbesondere Blätter, Blüten, Blütenteile, Rinde, Wurzeln, Rhizome, Früchte, Samen oder Gemische hiervon eingesetzt. Auch Pflanzensaft (z.B. Kokosmilch, Lakritze) und im Einzelfall auch wässrige, ölige oder alkoholische Auszüge können als Würzstoff verwendet werden.

Gemäß einer weiteren Ausführungsform können die Würzstoffe auch hinsichtlich ihrer Wirkung einklassifiziert werden. Insbesondere sind die Würzstoffe ausgewählt aus konservierenden Würzstoffen (z.B. Chili oder Rosmarin), Appetit anregenden Würzstoffen (z.B. aufgrund eines Bitterstoffs), verdauungsfördernden Würzstoffen (z.B. Pfeffer), Blähungen verhindernden Würzstoffen (z.B. Fenchel, Anis oder Kümmel), geschmacksverbessernden Würzstoffen (z.B. Rosenwasser oder Orangenblüten), geschmacksverstärkenden Würzstoffen (z.B. Vanille und im Einzelfall auch Glutamat oder Hefeextrakte), die Bildung von Gallenflüssigkeit und die Fettverdauung anregende Würzstoffen (z.B. Zwiebel und Knoblauch), für die Darmflora günstigen Würzstoffen (z.B. Ingwer und Knoblauch), das Herz-Kreislauf-System aktivierenden Würzstoffen oder Würzstoffen mit aphrodisierender Wirkung (Nelken, Chilli, Ingwer, Kakao(bohnen)), konzentrationsfördernden Würzstoffen (Kakao(bohnen), Kaffee(bohnen), Guarana, Colanuss) und entspannend oder beruhigend wirkenden Würzstoffen (Salbei, Muskat).

Für die Herstellung der Würzschäume werden insbesondere zerkleinerte (vor allem pulverisierte) Würzstoffe oder zerkleinerte (vor allem pulverisierte) Mischungen von Würzstoffen verwendet; es können aber auch wässrige, ölige oder alkoholische Auszüge eingesetzt werden.

Die pflanzlichen Würzstoffe können insbesondere ausgewählt werden aus Anis, Bärlauch, Basilikum, Beifuß, Bohnenkraut, Cardamom, Curcuma, Dill, Estragon, Fenchel, Galgant, Hopfen, Ingwer, Kaffee(bohnen), Kakao(bohnen), Kapern, Kerbel, Knoblauch, Kokos, Koriander, Kren (Meerrettich), Kreuzkümmel, Kümmel, Lavendel, Liebstöckel, Limette, Lorbeer, Majoran, Mohn, Muskatnuss, Muskatblüte, Nelken, Orange, Oregano, Paprika und Chili (z.B. in Form von Rosenpaprika, edelsüßem Paprika oder Cayennepfeffer), Petersilie, Pfeffer (z.B. grüner, schwarzer, weißer oder rosa Pfeffer), Pfefferminze, Pilze (z.B. Trüffel), Piment, Rose, Rosmarin, Safran, Salbei, Schnittlauch, Schwarzkümmel, Senf, Sternanis, Thymian, Vanille, Wacholder, Waldmeister, Wasabi, Ysop, Zimt, Zitrone, Zitronengras, Zitronenmelisse, Zwiebel und Gemischen von einem der vorgenannten Würzstoffe mit mindestens einem weiteren, insbesondere einem weiteren der vorstehend genannten Würzstoffe. Auch andere Früchte / Fruchtextrakte können generell als Würzstoffe dienen.

Unter einem Würzstoff sind üblicherweise kein Zucker und auch kein Kochsalz zu verstehen. Diese können aber neben dem eigentlichen Würzstoff im Würzschaum enthalten sein.

Gemäß einer Ausführungsform beträgt der Anteil des Würzstoff in dem in Schritt B) erhaltenen Gemisch ≤ 3 Gew.-%, insbesondere ≤ 1 Gew.-%. Als obere Grenze ist häufig die Löslichkeit des Würzstoffs in der Fettsäureesterkomponente ein Kriterium, wobei - wie ausgeführt - auch eine Verkapselung des Würzstoffs vorliegen kann. Grundsätzlich ist aber festzustellen, dass die erfindungsgemäßen Schäume nicht nur gewährleisten, dass (bei stark schmeckenden, scharf schmeckenden oder sehr teuren Stoffen) besonders geringe Konzentrationen des Würzstoffs aufgebracht werden können, sondern auch, dass überhaupt bestimmte Würzstoffe für Schaumapplikationen zugänglich sind. Durch den relativ hohen Fettsäureesteranteil kann nämlich auch ein besonders hoher Anteil schlechter löslicherer Würzstoffe in den Schaum eingebracht werden.

Gemäß einer weiteren Ausführungsform wird das in Schritt C) eingebrachte Gas zumindest teilweise ausgewählt aus Gasen, die fettlöslich sind. Insbesondere kann dieses fettlösliche Gas ausgewählt sein aus einem oder mehreren Gasen der Gruppe bestehend aus Stickstoff, Lachgas, Xenon und Kohlenwasserstoff-Gasen, insbesondere Butan und/oder Propan. Ferner sind generell Gase geeignet, die den Druck nachliefern. Das eingebrachte Gas kann entweder aus einem fettlöslichen Gas, insbesondere einem oder mehreren der vorstehend genannten Gase, bestehen oder diese enthalten. In letzterem Fall kann beispielsweise angereicherte Luft als Gas eingesetzt werden, wobei der Gehalt der fettlöslichen Gase im angereicherten Gas den Gehalt der fettlöslichen Gase in Luft zumindest um 10 Gew.-% (absolut) häufig um 15 Gew.-% und oft um 20 Gew.-% (absolut) übersteigt. Mit Hilfe derartiger fettlöslicher Gase kann die Schaumbildung verbessert werden.

Neben Fettsäureesterkomponente, Proteinkomponente, Würzstoff und Gas ist in dem erfindungsgemäßen Würzschaum bzw. der Zusammensetzung zu dessen Herstellung (in dem noch kein Gas enthalten ist) vor allem Wasser vorhanden. Der Wasseranteil der Zusammensetzung, die nach Schritt B) erhalten wird, beträgt 30 bis 95 Gew.-% (dementsprechend beträgt der aufsummierte Anteil von Proteinkomponente und Fettsäureesterkomponente 5 bis 70 Gew.-%, wenn man den Würzstoffgehalt in erster Näherung vernachlässigt). Häufig wird allerdings Wasser in einem Gesamtanteil von 65 bis 95 Gew.-% vorliegen, insbesondere im Bereich 60 bis 70 Gew.-%. (Der aufsummierte Fettsäureesterkomponente- / Proteinkomponente-Anteil beträgt dann - bei Vernachlässigung des Würzstoffanteils - 10 bis 35 %, insbesondere 20 bis 30 %; allerdings werden häufig der Würzstoffgehalt und der Gehalt weiterer Zusatzstoffe nicht vernachlässigbar sein, so dass der aufsummierte Anteil der beiden Komponenten im Regelfall niedriger ist und beispielsweise 10 bis 30 Gew.-%, insbesondere 15 bis 25 Gew.-% beträgt).

Statt Wasser kann grundsätzlich auch eine Mischung von verschiedenen Flüssigkeiten vorliegen (etwa eine Mischung von Wasser und Ethanol); im Regelfall liegt aber - abgesehen von gegebenenfalls flüssig vorliegender Proteinkomponente, Fettsäureesterkomponente und Würzstoffkomponente keine weitere Flüssigkeit als Wasser vor.

Gemäß einer weiteren Ausführungsform werden in Schritt B) neben Proteinkomponente und Fettsäureesterkomponente mit Würzstoff sowie Wasser auch noch Hilfsstoffe zugemischt. Als typische Hilfsstoffe sind insbesondere Verdicker, Polysaccharide und Konservierungsmittel zu nennen.

Als Verdicker kommen beispielsweise Celluloseether (z.B. Methylcellulose), modifizierte Stärke, Guaran, Carrageene, also insbesondere Polysaccharidderivate wie Polysaccharidether und -ester in Betracht. Ferner sind Johannisbrotkernmehl und andere in der Lebensmittelindustrie verwendete Verdicker zu nennen. Mittels der Verdicker kann eine Erhöhung der Stabilität der Schäume realisiert werden. Gemäß einer Ausführungsform kann der schaumstabilisierende Bestandteil somit auch einerseits aus einem in der Proteinkomponente enthaltenen schaumstabilisierenden Protein und/oder einem in der Fettsäureesterkomponente enthaltenen schaumstabilisierenden Bestandteil und andererseits aus dem Verdicker, insbesondere einem der zuvor explizit aufgeführten Verdicker, bestehen.

Als Konservierungsmittel ist beispielsweise Vitamin E-Acetat zu nennen, das einen Oxidationsschutz bietet.

Nicht als Hilfsstoffe bzw. generell nicht enthalten sind üblicherweise - wie bereits ausgeführt - synthetische Tenside. Auch auf den Zusatz teilsynthetischer Tenside wird üblicherweise verzichtet. Natürliche Tenside können zwar enthalten sein, im Regelfall werden diese aber nicht absichtlich zugesetzt, sondern sind im Regelfall nur in der Fettsäureesterkomponente und/oder der Proteinkomponente enthalten.

Bezüglich des Kohlenhydratanteils, der zwar grundsätzlich - wie ausgeführt - vorliegen kann, ist zu beachten, dass der Anteil von Kohlenhydraten, die in der Lebensmittelindustrie zum Süßen eingesetzt werden, üblicherweise vernachlässigbar ist (also bezogen auf das Gemisch das nach Schritt B) erhalten wird, kleiner 1 Gew.-% ist. Generell ist der Anteil von Kohlenhydraten normalerweise geringer als der Gewichtsanteil der Proteinkomponente, insbesondere auch geringer als der des Proteinanteils der Protein komponente.

Der Anteil von zusätzlichen Hilfsstoffen an dem Gemisch, dass nach Schritt B) erhalten wird kann insbesondere 0 bis 20 Gew.-% betragen, liegt üblicherweise aber im Bereich 2 bis 5 Gew.-%.

Im Folgenden wird das erfindungsgemäße Verfahren - ohne Einschränkung der Allgemeinheit - noch näher beschrieben:
Für den Würzschaum wird zunächst die Proteinkomponente, die insbesondere so ausgewählt wird, dass sie den Würzschaum später stabilisieren kann, in Wasser homogenisiert. Als Proteinkomponente können beispielsweise Weizenprotein-, Reisprotein-, Sojaprotein-, Kartoffelprotein-, Rapsprotein und Milchprotein-haltige Proteinkomponenten verwendet werden. Diese Proteine können dann gegebenenfalls zur Verbesserung ihrer Eigenschaften chemisch modifiziert oder auch hydrolysiert oder zerkleinert werden. Grundsätzlich kann auch ein bereits wässrig vorliegendes Protein bzw. eine wässrig vorliegende Proteinkomponente Verwendung finden, wobei dann für die vorstehenden Angaben der Gewichtsprozente zunächst der Wassergehalt zu bestimmen und abzuziehen ist.

Im nächsten Schritt werden zu dem Homogenisat aus Proteinkomponente und Wasser, die ggf. zuzugebenden Hilfsstoffe, beispielsweise ein Verdicker und ein Würzstoff und Konservierungsmittel wie Vitamin-E-Acetat zugegeben und ebenfalls homogen verteilt. Schließlich wird noch die Fettsäureesterkomponente, insbesondere ein Öl, zu der gut homogenisierten Mischung hinzugegeben und verteilt. Der Würzstoff kann bereits in dieser Fettsäureesterkomponente vorliegen, kann aber auch in das Gemisch eingebracht werden bzw. bereits zu einem früheren Zeitpunkt eingebracht sein. Nachdem es sich häufig allerdings um lipophile Stoffe handeln wird, wird üblicherweise der Würzstoff zusammen mit der Fettsäureesterkomponente dem Gemisch zugegeben.

Um aus dem vorstehend beschriebenen Gemisch einen Schaum zu generieren wird die Emulsion beispielsweise mit einem möglichst fettlöslichen Treibgas in einem Druckgasgefäß unter Druck gesetzt. Das Gas kann dabei insbesondere unter einem Druck von 1 bis 15 hPa, bevorzugt 5 bis 9 hPa eingebracht werden. Der fertige Schaum kann üblicherweise nach kräftigem Schütteln aus dem Druckgasgefäß entnommen werden bzw. aus diesem aufgebracht werden.

Insbesondere auch das Einbringen des Gases ist nicht auf die bevorstehend beschriebene Art beschränkt, vielmehr sind auch andere Verfahren denkbar, die dem Fachmann bekannt sind.

Die Erfindung betrifft auch eine Zusammensetzung, die zur Herstellung des Würzschaums dienen kann, und die insbesondere nur noch das Gas eingebracht werden muss. Diese Zusammensetzung umfasst eine Fettsäureesterkomponente, eine Proteinkomponente, einen Würzstoff und Wasser, wobei die Fettsäureesterkomponente und/oder die Proteinkomponente einen schaumstoffstabilisierenden Bestandteil enthalten, der ein in der Proteinkomponente enthaltenes schaumstabilisierendes Protein und/oder einen in der Fettsäureester enthaltenen schaumstabilisierenden Bestandteil umfasst oder daraus besteht. In der Fettsäureesterkomponente liegt der Würzstoff, üblicherweise in gelöster oder verkapselter Form vor. Zumindest die Fettsäureesterkomponente ist dabei im Wesentlichen biogenen Ursprungs. Ferner liegen die Proteinkomponente und die Fettsäureesterkomponente in einem Gewichtsverhältnis von 1 : 10 bis 9 : 1 vor. Schließlich beträgt das Gewichtsverhältnis zwischen Wasser und den aufsummierten Anteilen der Proteinkomponente und der Fettsäureesterkomponente 1 : 1 bis 10:1 (Wasser liegt also im gewichtsmäßigen Überschuss vor. Der Anteil der Fettsäureesterkomponente in der Zusammensetzung beträgt üblicherweise mehr als 10 Gew.-%. Mit der erfindungsgemäßen Zusammensetzung ist es in einfacher Weise möglich, Würzschäume zu erzeugen, die für verschiedenste Anwendungen, insbesondere für das Marinieren geeignet sind.

Die vorliegende Erfindung betrifft schließlich auch einen Würzschaum, der gemäß den näher beschriebenen Verfahren herstellbar ist, bzw. durch Einbringen eines Gases in die vorstehend beschriebene Zusammensetzung erhältlich ist. Der Würzschaum hat üblicherweise ein glänzendes Erscheinungsbild und weist eine relativ hohe Schaumstoffstabilität auf, so dass vielfach ein Zerfließen des Schaumes frühestens nach zwanzig Minuten zu beobachten ist.

Gemäß einer Ausführungsform ist der Volumenanteil der nicht gasförmigen Bestandteile im frisch hergestellten Schaum maximal 35%, insbesondere 10 bis 35 %, beispielsweise 20 bis 30%. Es wird also ein Schaum erhalten, der gegenüber der noch nicht aufgeschäumten Formulierung zumindest ein etwa 3 mal größeres Volumen hat, so dass dementsprechend auch eine deutlich verbesserte Applizierbarkeit des Schaums gegenüber der gasfreien Formulierung gegeben ist.

Die erfindungsgemäßen Würzschäume können in der Nahrungsmittelindustrie in vielseitiger Art und Weise Verwendung finden. Als zentraler Aspekt steht im Vordergrund, dass keine synthetischen Materialien sondern im Wesentlichen biogene Materialien Einsatz finden und somit grundsätzlich eine Unbedenklichkeit für den Verzehr vorliegen sollte. Auf Grund der Schaumstruktur ist ferner zu konstatieren, dass ein Würzmittel auch in geringen Mengen homogen und gleichmäßig aufgebracht werden kann. Dies spielt insbesondere bei sehr scharf schmeckenden Würzmitteln oder sehr teuren Würzmitteln eine große Rolle.

Als Anwendungszweck derartiger Würzschäume sind beispielsweise Marinaden oder Schaumstrukturen, die insbesondere für die optische Darreichungsform wesentlich sind, zu nennen. Ferner sind Konservierungsschäume zu nennen, beispielsweise als Ersatz für Öle bei in Öl eingelegten Lebensmitteln. Beispielsweise ist bei eingelegten Oliven der Ersatz von gewürzhaltigem Olivenöl durch einen erfindungsgemäßen Würzschaum auf Olivenölbasis denkbar.

Ferner ist für die Würzanwendungen festzustellen, dass hierbei durch den erhöhten Fettsäureesterkomponenteanteil eine ausreichende Verteilung und ein ausreichender Transport der Geschmackskomponenten gewährleistet. Während für herkömmliche Marinaden in der Regel eine fertige Öl-Gewürzmischung verwendet wird oder eine pulverförmige Gewürzmischung mit mehreren Esslöffeln Öl angerührt werden muss, kann mit einem Würzschaum im Sinne der Erfindung ein vergleichbares Produkt aus weniger Fett bzw. Öl hergestellt werden. Das Öl lässt sich viel feiner dosieren und verteilt sich besser in den Poren, so dass einerseits eine bessere Marinierung des zu marinierenden Gutes erreicht wird und gleichzeitig eine deutliche Fett- und somit Kalorienreduzierung gegenüber herkömmlichen Marinaden möglich ist. Auch bei der Verwendung des Würzschaums in Vinaigretten, als Dip oder zur Verfeinerung von Suppen, Saucen etc. ist eine deutliche Kalorienreduzierung gegenüber der Nutzung herkömmlicher Fette wie Öl, Butter, Creme Fraiche etc. gegeben.

Außerdem besteht anders als bei Würzölen die Möglichkeit, den Würzschaum zu dekorativen Zwecken wie z. B. Schaumhaube auf Fleisch-Stücken oder Suppen etc. zu verwenden. Weitere Vorteile und vorteilhafte Ausführungsformen und Weiterbildungen der Erfindung ergeben sich im Folgenden - ohne Einschränkung der Allgemeinheit - aus den Beispielen.

### Beispiel 1

Der Würzschaum wird aus folgenden Bestandteilen zusammengesetzt:

| | |
|---|---|
| 30 g | Total Milk Protein (Firma Milei GmbH mit einem Proteingehalt von 80 % in der Trockenmasse, einem Lactosegehalt von 9%, einem Mineraliengehalt von 7%, einem Fettgehalt von 1 %, einem Wassergehalt von 6% (jeweils Gew.-%) und einem pH-Wert von 6,9) |
| 200 g | Wasser, bidestilliert |
| 5 g | Hydroxymethylpropylcellulose (4000 Tegocelfluid der Firma Evonik) |
| 5 g | Vitamin-E-Acetat |
| 50 g | Sonnenblumenöl der Marke Thomy |
| 1 ml | Mandarinenöl (als Würzstoff) |
| 8 g | Lachgas |

Das Protein wird eingewogen, Wasser hinzugegeben und die Mischung homogenisiert. Anschließend wird der Verdicker zugesetzt und es erfolgt eine erneute Homogenisierung. Schließlich werden die verbleibenden Komponenten, Vitamin E-Acetat (als Antioxidans), Sonnenblumenöl und Mandarinenöl zugemischt.

Die erhaltene Emulsion wird in ein Druckgefäß gegeben und mit 8 g Lachgas unter Druck gesetzt. Nach kräftigem Schütteln und einer kurzen Ruhezeit kann der fertige Schaum dem Druckbehälter entnommen werden. Der Volumenanteil der nicht gasförmigen Bestandteile beträgt etwa 25 %.

Der erhaltene Schaum ist weiß und besitzt einen starken Geruch nach Mandarinenöl.

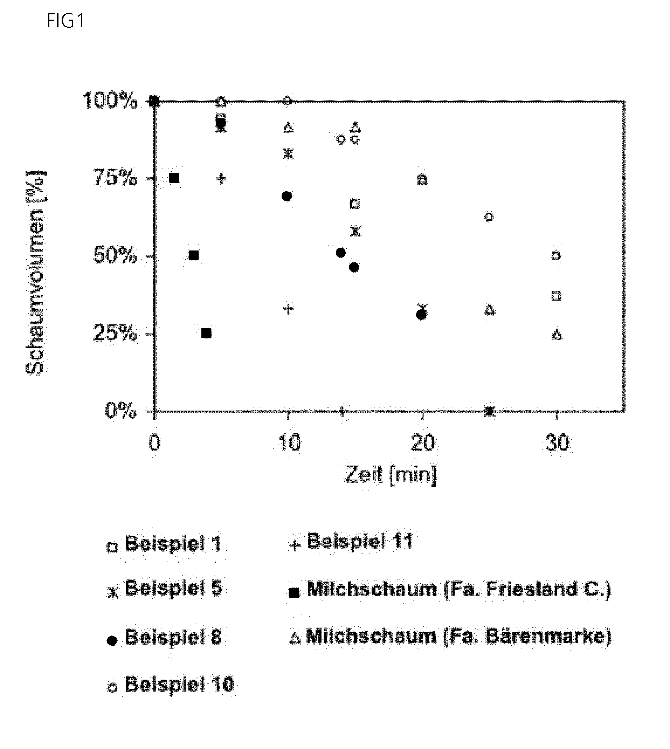

In einem Drainageversuch wurde der erfindungsgemäße Schaum mit einem kommerziell erhältlichem Chantibic - Milchschaum mit 0 % Fett (Firma Friesland Campina. Lumen / Belgien) verglichen, der nur Verdicker (Xanthan) aber keinen Emulgator enthält. Figur 1 zeigt die zeitliche Abnahme des Schaumvolumens, wobei der Schaum nach Beispiel 1 (ungefüllte Quadrate) eine deutlich höhere Stabilität als das käufliche Produkt (gefüllte Quadrate) aufweist. Die erfindungsgemäße Öl / Protein-Mischung weist also gegenüber dem kommerziellen Produkt mit einem wesentlich niedrigeren Fett-Gehalt deutliche Vorteile auf. Vergleichbare Stabilitäten werden nur mit einem kommerziell erhältlichem Milchschaum (Fa. Bärenmarke) erzielt, der allerdings zusätzlich Emulgatoren (E471, E472) und verschiedene Verdicker enthält (Dreiecke).

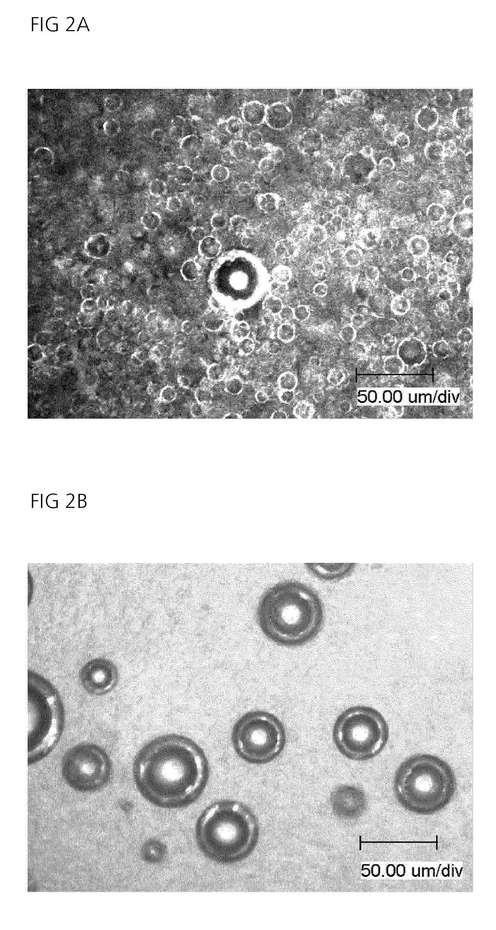

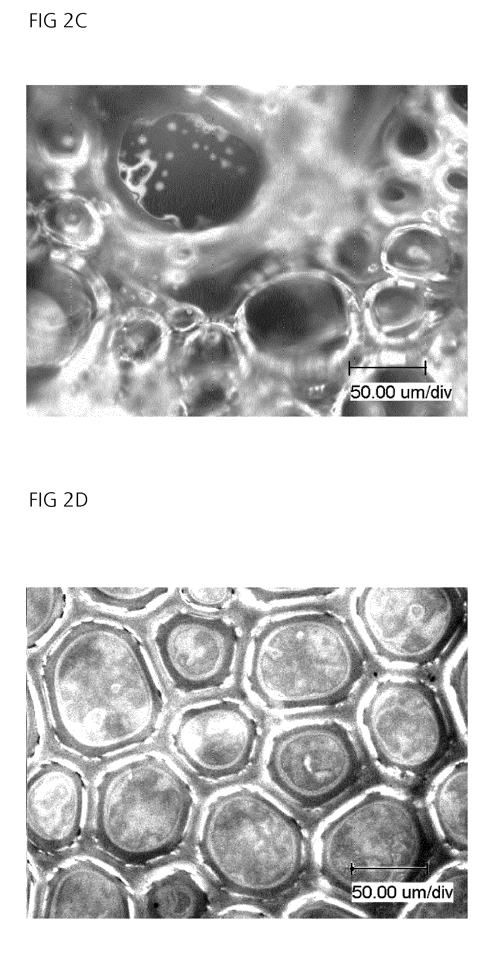

Die Figuren 2A bis 2D zeigen lichtmikroskopische Aufnahme verschiedener Schäume. Fig 2A zeigt den erfindungsgemäßen Schaum, Fig 2B den fettfreien Milchschaum der Firma Friesland Campina aus Figur 1, und ferner Schäume, die nicht dem Nahrungsmittelsektor zuzurechnen sind, nämlich in Fig 2C einen mittels eines modifizierten Proteins stabilisierten Schaum (Fußpflegeschaum der Firma Allpresan) und Fig 2D einen Trockenschaum (Rasierschaum der Marke Insana, Firma Rossmann - enthält synthetische Tenside). Auf Grund des verwendeten Objektträgers wurden insbesondere in Fig 2A und Fig 2B Gasblasen in gewissem Umfang zerstört, es lässt sich allerdings sehr schön in Fig. 2A erkennen, dass eine besonders homogene Verteilung der Fettkomponente im Schaum vorliegt. Die Größe der Fett "tröpfchen" ist heterogen und reicht von 0,5 bis 30 µm (Durchmesser). Der mittlere Durchmesser der Fett "tröpfchen beträgt etwa 5 bis 15 µm (ermittelt durch Auswertung der lichtmikroskopischen Aufnahme).

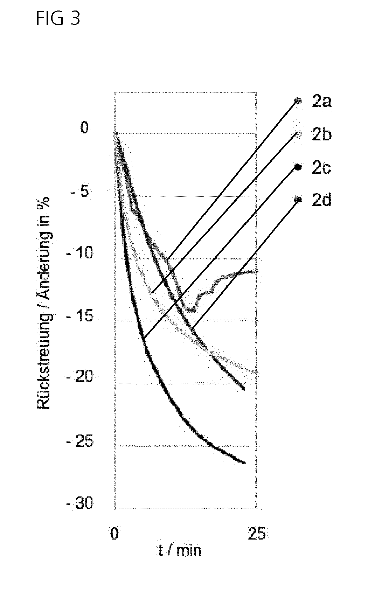

Lichtstreuexperimente erlauben eine Aussage über die Schaumstabilität. Eine Verringerung der Rückstreuintensität ist auf eine Koagulation der Gasblasen zurückzuführen. Figur 3 zeigt die Abnahme der Rückstreuintensität in Abhängigkeit der Zeit bezogen auf die Ausgangsintensität für die Schäume gemäß Fig. 2A bis 2D, wobei 2a den Schaum gemäß Fig 2A bezeichnet, 2b den Schaum gemäß Fig 2B, 2c den Schaum gemäß Fig 2C und 2d den Schaum gemäß Fig 2D.

### Beispiel 2

Der Schaum enthält folgende Bestandteile

| | |
|---|---|
| 50 g | Sprühmagermilchpulver, aufkonzentriert (Firma Nöll & Co. GmbH, Büren mit einem Milchzuckergehalt von ca. 52 %, Proteingehalt von mindestens 34%, Milchmineraliengehalt von ca. 7,5 - 8,0 %, Milchfettgehalt von max. 1,0 %, Wassergehalt von max. 5,0 % (jeweils Gew.-%) und einem pH-Wert von ca. 6,6 |
| 200g | Wasser, bidestilliert |
| 3 g | Guaran |
| 30 g | Sonnenblumenöl der Marke Thomy |
| 0,5 ml | Chili-Öl |
| 8 g | Lachgas |

Die Zusammensetzung wird wie im Beispiel 1 verarbeitet; man erhält ebenfalls einen Schaum mit deutlich erhöhter Stabilität. Im Vergleich zu Beispiel 1 ist hier der Ölanteil etwas erniedrigt und der Proteinanteil etwas erhöht; die erzielten Ergebnisse sind aber vergleichbar.

### Beispiel 3

Der Schaum enthält folgende Bestandteile

| | |
|---|---|
| 30 g | Weizenprotein, desaminiert (Firma Cargill Company) |
| 200 g | Wasser, bidestilliert |
| 5 g | modifizierte Stärke |
| 5 g | Vitamin-E-Acetat |
| 50 g | Sonnenblumenöl der Marke Thomy |
| 1 ml | Citronellaöl (als Würzstoff) |
| 8 g | Lachgas |

Die Zusammensetzung wird wie im Beispiel 1 verarbeitet; man erhält einen Schaum mit gegenüber dem Schaum aus Beispiel 1 deutlich erhöhter Stabilität aber vergleichbarer Schaumstruktur. Der erhaltene Schaum ist leicht braun und besitzt einen citrusartigen Geruch.

### Beispiele 5 bis 11

In den nachfolgenden Beispielen wurde der Einfachheit halber vielfach auf den Zusatz eines Würzstoffs verzichtet. Es versteht sich von selbst, dass sich hierdurch keine wesentlichen Unterschiede hinsichtlich der Eigenschaften der erhaltenen Schäume ergeben. Im Übrigen ist ohne einen Würzstoff wie z.B. Chiliöl eine bessere Beurteilung des Geruchs des erhaltenen Schaums möglich.

### Beispiel 5

Der Schaum enthält folgende Bestandteile

| | |
|---|---|
| 30 g | Total Milk Protein wie in Beispiel 1 definiert |
| 200 g | Wasser, bidestilliert |
| 0,9 g | Hydroxymethylpropylcellulose |
| 50 g | Sonnenblumenöl der Marke Horeca |
| 16 g | Lachgas |

Beispiel 5 unterscheidet sich von Beispiel 1 dahingehend, dass deutlich weniger Verdicker verwendet wurde und mit der doppelten Menge Gas aufgeschäumt wurde. Darüber hinaus wurde auf den Wirkstoff und die maskierende Komponente verzichtet. Im Vergleich zum Beispiel 1 ist die Hydroxymethylpropylcellulose nicht vorformuliert und wird wie folgt angesetzt: 20 ml des Wassers werden auf 80°C erhitzt und die Hydroxypropylmethylcellulose langsam unter ständigem Rühren hinzugeben bis sie vollständig gelöst ist, weitere 30 ml Wasser hinzugegeben und 30 min nachgerührt.

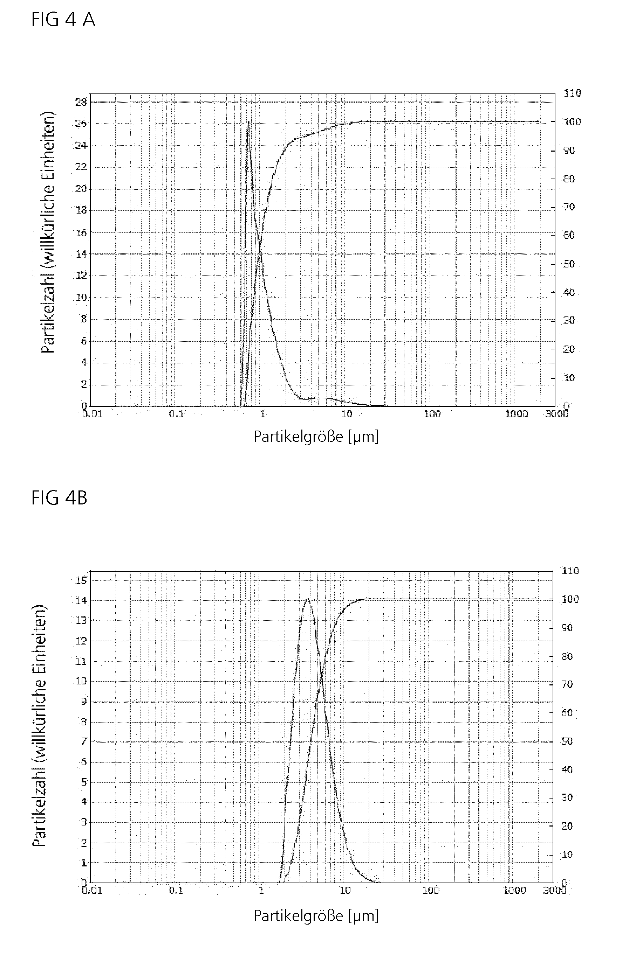

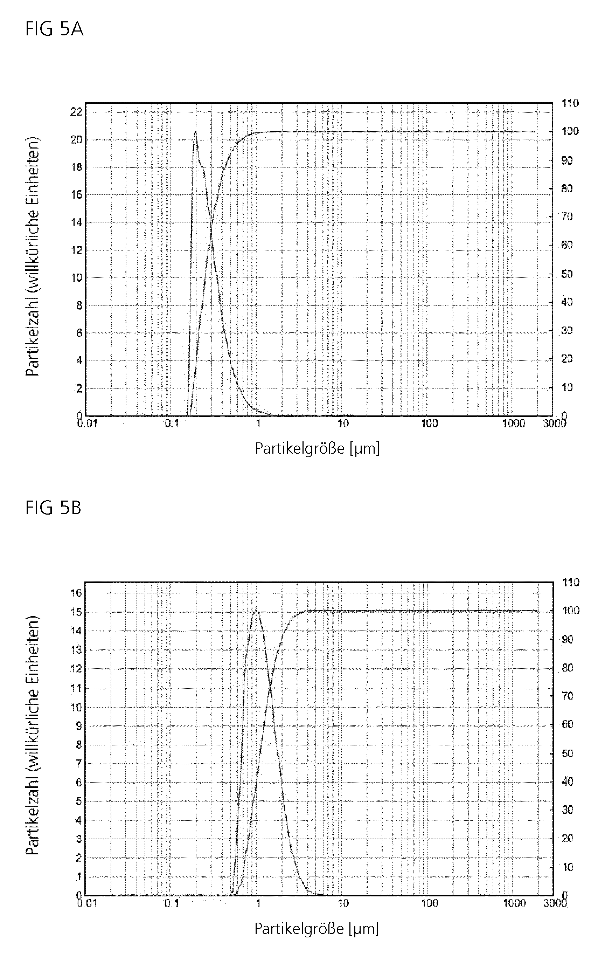

Das Protein wird eingewogen, 150 ml Wasser, das Sonnenblumenöl und die vorformulierte Hydroxymethylpropylcellulose hinzugegeben und die Mischung homogenisiert. Figur 4A zeigt die mittels dynamischer Lichtstreuung ermittelte Partikelgrößenverteilung (d.h. die Tröpfchengrößen der Fettsäureesterkomponente) für die erhaltene Emulsion. Die mittlere Partikelgröße beträgt 9,5 µm und ist damit größer als in Sprühsahne (1,9 µm - Marke Milboa der Firma Lidl), für die die Partikelgrößenverteilung in Figur 5A gezeigt ist. Milchschaum (Firma Bärenmarke) hat ebenfalls eine kleinere mittlere Partikelgröße (2,8 µm -Partikelgrößenverteilung in Figur 5B). Die erhaltene Emulsion wird in ein Druckgefäß gegeben und mit 16 g Lachgas unter Druck gesetzt. Nach kräftigem Schütteln und einer kurzen Ruhezeit kann der fertige Schaum dem Druckbehälter entnommen werden. Der Volumenanteil der nicht gasförmigen Bestandteile beträgt etwa 12 %.

Man erhält einen Schaum mit gegenüber dem Schaum aus Beispiel 1 vergleichbarer Schaumstruktur. Der erhaltene Schaum ist weiß und besitzt einen leichten Milch-Geruch. Der höhere Gasanteil führt zu einem leichteren Schaum. Figur 1 (Sterne) zeigt eine etwas geringere Schaumstabilität als bei Beispiel 1.

### Beispiel 6

Der Schaum enthält folgende Bestandteile

| | |
|---|---|
| 30 g | Total Milk Protein wie in Beispiel 1 definiert |
| 200 g | Wasser, bidestilliert |
| 0,9 g | Hydroxymethylpropylcellulose |
| 100 g | Sonnenblumenöl der Marke Horeca |
| 16 g | Lachgas |

Beispiel 6 unterscheidet sich von Beispiel 5 dahingehend, dass die doppelte Menge Sonnenblumenöl verwendet wurde. Die Zusammensetzung wird wie im Beispiel 5 verarbeitet; man erhält einen Schaum mit gegenüber dem Schaum aus Beispiel 5 vergleichbarer Stabilität, Schaumstruktur und Gasvolumenanteil. Der erhaltene Schaum ist weiß und besitzt einen leichten Milch-Geruch. Der Volumenanteil der nicht gasförmigen Bestandteile beträgt etwa 12 %. Figur 4B zeigt die Partikelgrößenverteilung für die dem Schaum zugrundeliegende Emulsion. Die mittlere Partikelgröße beträgt 7,8 µm und ist kleiner als bei Beispiel 5.

### Beispiel 7

Der Schaum enthält folgende Bestandteile

| | |
|---|---|
| 30 g | Total Milk Protein wie in Beispiel 1 definiert |
| 200 g | Wasser, bidestilliert |
| 0,9 g | Hydroxymethylpropylcellulose |
| 25 g | Natives Olivenöl Extra der Marke Primadonna |
| 16 g | Lachgas |

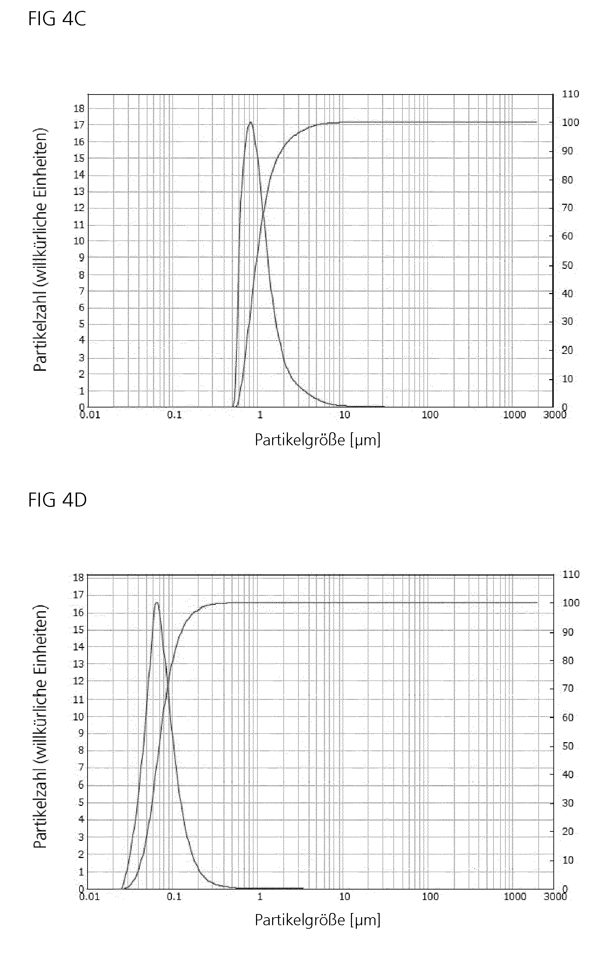

Beispiel 7 unterscheidet sich von Beispiel 5 dahingehend, dass nur die halbe Menge Öl und zwar Olivenöl statt Sonnenblumenöl verwendet wurde. Die Zusammensetzung wird wie im Beispiel 5 verarbeitet; man erhält einen Schaum mit gegenüber dem Schaum aus Beispiel 5 vergleichbarer Stabilität, Schaumstruktur und Gasvolumenanteil. Der erhaltene Schaum ist weiß/leicht grünlich und besitzt keinen markanten Eigengeruch. Figur 4C zeigt die Partikelgrößenverteilung für die dem Schaum zugrundeliegende Emulsion. Die mittlere Partikelgröße beträgt 5,6 µm ist deutlich kleiner als bei Beispiel 5.

### Beispiel 8

Der Schaum enthält folgende Bestandteile

| | |
|---|---|
| 30 g | Total Milk Protein wie in Beispiel 1 definiert |
| 200 g | Wasser, bidestilliert |
| 5 g | Hydroxymethylpropylcellulose (4000 Tegocelfluid der Firma Evonik) |
| 5 g | Vitamin-E-Acetat |
| 50 g | Rapskernöl "Vielseitig" der Marke Teutoburger Ölmühle |
| 20 Tropfen | Mandarinenöl (als Würzstoff) |
| 8 g | Lachgas |

Die Zusammensetzung wird wie im Beispiel 1 verarbeitet; man erhält einen Schaum mit gegenüber dem Schaum aus Beispiel 1 vergleichbarer Schaumstruktur und etwas geringerer Stabilität (Figur 1 - gefüllte Kreise). Der erhaltene Schaum ist beige und besitzt einen leichten Milch-Geruch. Figur 4D zeigt die Partikelgrößenverteilung für die dem Schaum zugrundeliegende Emulsion. Die mittlere Partikelgröße beträgt 0,5 µm und ist damit deutlich kleiner als in den vorhergehenden Beispielen 5 bis 7.

### Beispiel 9

Der Schaum enthält folgende Bestandteile

| | |
|---|---|
| 30 g | Weizenprotein, natur (Firma Cargill Company) |
| 200 g | Wasser, bidestilliert |
| 5 g | Hydroxymethylpropylcellulose (4000 Tegocelfluid der Firma Evonik) |
| 5 g | Vitamin-E-Acetat |
| 50 g | Rapskernöl "Vielseitig" der Marke Teutoburger Ölmühle |
| 20 Tropfen | Mandarinenöl (als Würzstoff) |
| 8 g | Lachgas |

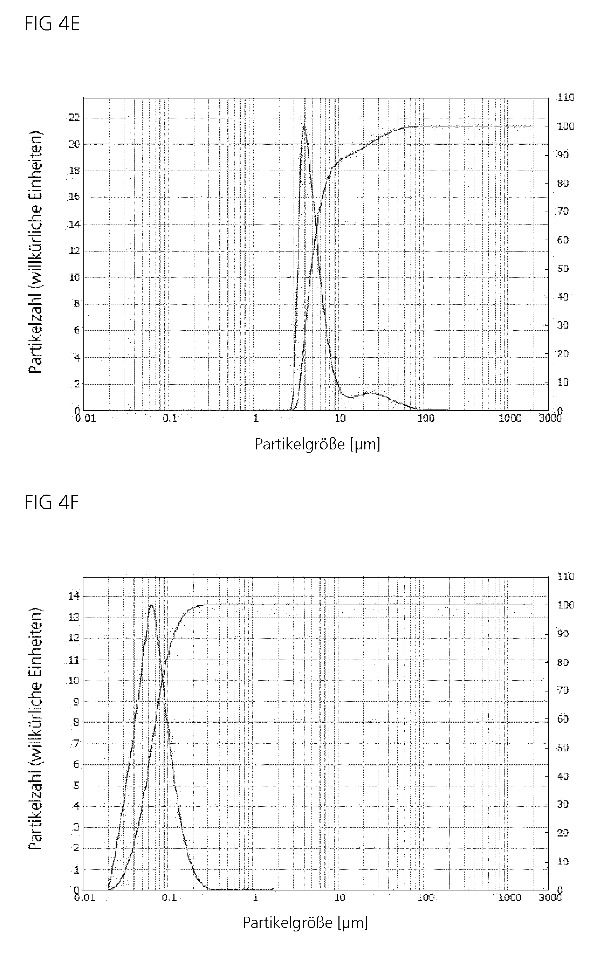

Die Zusammensetzung wird wie im Beispiel 1 verarbeitet; man erhält einen Schaum mit gegenüber dem Schaum aus Beispiel 1 vergleichbarer Schaumstruktur. Der erhaltene Schaum ist beige und besitzt einen deutlichen Weizenprotein-Geruch. Der Volumenanteil der nicht gasförmigen Bestandteile beträgt etwa 22 %. Figur 4E zeigt die Partikelgrößenverteilung für die dem Schaum zugrundeliegende Emulsion. Die mittlere Partikelgröße beträgt 51,6 µm.

### Beispiel 10

Der Schaum enthält folgende Bestandteile

| | |
|---|---|
| 30 g | Weizenprotein, desaminiert (Firma Cargill Company) |
| 200 g | Wasser, bidestilliert |
| 3 g | Hydroxymethylpropylcellulose (4000 Tegocelfluid der Firma Evonik) |
| 5 g | Vitamin-E-Acetat |
| 50 g | Rapskernöl "Vielseitig" der Marke Teutoburger Ölmühle |
| 20 Tropfen | Mandarinenöl (als Würzstoff) |
| 8 g | Lachgas |

Beispiel 10 unterscheidet sich von Beispiel 9 dahingehend, dass weniger Verdicker verwendet wurde. Die Zusammensetzung wird wie im Beispiel 1 verarbeitet; man erhält einen Schaum mit gegenüber dem Schaum aus Beispiel 1 vergleichbarer Schaumstruktur und sogar etwas erhöhter Stabilität (Figur 1 - ungefüllte Kreise). Der erhaltene Schaum ist beige und besitzt einen deutlichen Weizenprotein-Geruch. Figur 4F zeigt die Partikelgrößenverteilung für die dem Schaum zugrundeliegende Emulsion. Die mittlere Partikelgröße beträgt 0,2 µm und ist deutlich kleiner als bei Beispiel 9.

### Beispiel 11

Der Schaum enthält folgende Bestandteile

| | |
|---|---|
| 30 g | Total Milk Protein wie in Beispiel 1 definiert |
| 200 g | Wasser, bidestilliert |
| 0,9 g | Hydroxymethylpropylcellulose |
| 50 g | Sonnenblumenöl der Marke Horeca |
| 8 g | Lachgas |

Beispiel 11 unterscheidet sich von Beispiel 5 dahingehend, dass weniger Gas zum Aufschäumen verwendet wurde. Die Zusammensetzung wird wie im Beispiel 1 verarbeitet; man erhält einen Schaum mit gegenüber dem Schaum aus Beispiel 1 vergleichbarer Schaumstruktur und Stabilität (Figur 1). Der erhaltene Schaum ist beige und besitzt einen deutlichen Weizenprotein-Geruch.

Die Zusammensetzung wird wie im Beispiel 5 verarbeitet; man erhält einen Schaum (Figur 1, Kreuze) mit gegenüber dem Schaum aus Beispiel 5 geringerer Stabilität. Der erhaltene Schaum ist weiß und besitzt einen leichten Milch-Geruch. Der Volumenanteil der nicht gasförmigen Bestandteile beträgt etwa 25 %. Figur 4A zeigt die Partikelgrößenverteilung.

Die Erfindung ist nicht durch die Beschreibungen an Hand der Ausführungsbeispiele beschränkt. Vielmehr umfasst die Erfindung jedes neue Merkmal sowie jede Kombination von Merkmalen, insbesondere jede Kombination von Patentansprüchen beinhaltet, auch wenn dieses Merkmal oder diese Kombination selbst nicht explizit in den Patentansprüchen oder Ausführungsbeispielen angegeben ist.

## Patentansprüche

1. Verfahren zur Herstellung eines Würzschaums, der ein Protein sowie ein Fett und/oder ein Wachs umfasst, mit folgenden Schritten:
A) Bereitstellung des Fetts und/oder des Wachses (Fettsäureesterkomponente) und der Proteinkomponente, wobei
- die Fettsäureesterkomponente und/oder die Proteinkomponente einen schaumstabilisierenden Bestandteil enthalten, der ein in der Proteinkomponente enthaltenes schaumstabilisierendes Protein und/oder einen in der Fettsäureesterkomponente enthaltenen schaumstabilisierenden Bestandteil umfasst oder daraus besteht,
- zumindest die Fettsäureesterkomponente im Wesentlichen biogenen Ursprungs ist,
- die Proteinkomponente und die Fettsäureesterkomponente in einem Gewichtsverhältnis von 1:10 bis 9:1 vorliegen,
- in die Fettsäureesterkomponente mindestens ein Würzstoff eingebracht ist oder eingebracht wird,
B) Mischen der Proteinkomponente und der Fettsäureesterkomponente gegebenenfalls unter Zusatz von Wasser, so dass Wasser und die summierten Anteile von Proteinkomponente und Fettsäureesterkomponente in einem Gewichtsverhältnis von 1:1 bis 10:1 vorliegen und wobei der Anteil der Fettsäureesterkomponente im entstandenen Gemisch größer 10 Gew.-% ist,
C) Einbringen eines Gases in das nach Schritt B) erhaltene Gemisch, so dass ein Schaum entsteht.

2. Verfahren nach dem vorhergehenden Anspruch, wobei die Fettsäureesterkomponente, die Proteinkomponente und der schaumstabilisierende Bestandteil im Wesentlichen biogenen Ursprungs ist

3. Verfahren nach einem der vorhergehenden Ansprüche, wobei nach Schritt B) eine Emulsion erhalten wird.

4. Verfahren nach einem der vorhergehenden Ansprüche, wobei die Fettsäureesterkomponente ein Öl oder ein Wachs ist, das bei 37 °C flüssig ist.

5. Verfahren nach einem der vorhergehenden Ansprüche, wobei der Anteil der Fettsäureesterkomponente in dem in Schritt B) erhaltenen Gemisch 10 bis 50 Gew.-% beträgt, und insbesondere zwischen 10 und 35 Gew.-% liegt.

6. Verfahren nach einem der vorhergehenden Ansprüche, wobei die Proteinkomponente ein Protein und/oder ein Proteinextrakt umfasst.

7. Verfahren nach einem der vorhergehenden Ansprüche, wobei die Proteinkomponente ein pflanzliches Protein, insbesondere ein Getreideprotein, ein Hülsenfruchtprotein oder ein Kartoffelprotein, ein Eierprotein, ein Rapsprotein oder ein Milchprotein umfasst oder daraus besteht.

8. Verfahren nach einem der vorhergehenden Ansprüche, wobei der Würzstoff in gelöster Form oder in verkapselter Form vorliegt.

9. Verfahren nach einem der vorhergehenden Ansprüche, wobei der Würzschaum zusätzlich ein Kohlenhydrat enthält und der Kohlenhydryatanteil < 1 Gew.-% beträgt.

10. Verfahren nach einem der vorhergehenden Ansprüche, wobei der Anteil des Würzstoffs in dem in Schritt B) erhaltenen Gemisch ≤ 3 Gew.-%, insbesondere ≤ 1 Gew.-% beträgt.

11. Verfahren nach einem der vorhergehenden Ansprüche, wobei das eingebrachte Gas zumindest zum Teil ein fettlösliches Gas ist, das insbesondere ausgewählt ist aus Stickstoff, Lachgas, Xenon und Gemischen hiervon.

12. Verfahren nach einem der vorhergehenden Ansprüche, wobei in Schritt B) zusätzlich Hilfsstoffe, insbesondere Verdicker und/oder Konservierungsmittel zugemischt werden.

13. Zusammensetzung zur Herstellung eines Würzschaums umfassend eine Fettsäureesterkomponente, eine Proteinkomponente, einen Würzstoff und Wasser wobei
- die Fettsäureesterkomponente und/oder die Proteinkomponente einen schaumstabilisierenden Bestandteil enthalten, der ein in der Proteinkomponente enthaltenes schaumstabilisierendes Protein und/oder einen in der Fettsäureesterkomponente enthaltenen schaumstabilisierenden Bestandteil umfasst oder daraus besteht,
- die Fettsäureesterkomponente den Würzstoff enthält,
- zumindest die Fettsäureesterkomponente im Wesentlichen biogenen Ursprungs ist,
- die Proteinkomponente und die Fettsäureesterkomponente in einem Gewichtsverhältnis von 1:10 bis 10:1 vorliegen,
- das Wasser und die summierten Anteile von Proteinkomponente und Fettsäureesterkomponente und in einem Gewichtsverhältnis von 1:1 bis 20:1 vorliegen und wobei der Anteil der Fettsäureesterkomponente im entstandenen Gemisch größer 10 Gew.-% beträgt.

14. Würzschaum herstellbar mit dem Verfahren nach einem der Ansprüche 1 bis 12 und/oder durch Einbringen eines Gases in die Zusammensetzung gemäß Anspruch 13.

15. Schaum nach dem vorhergehenden Anspruch, wobei der Anteil nicht gasförmiger Bestandteile maximal35 Vol% beträgt.
